# FASCICULE DE BREVET EUROPEEN

(11) **EP 1 208 861 B1**
(45) Date de publication et mention de la délivrance du brevet: **11.05.2005**
(21) Numéro de dépôt: 01402942.5
(22) Date de dépôt: 16.11.2001
(51) Int. Cl.: A61M 5/32, A61M 5/00

(54) **Dispositif de protection pour aiguille de seringue**
Schutzvorrichtung für eine Spritzennadel
Protecting device for a syringe needle

(30) Priorité: 17.11.2000 FR 0014843
(43) Date de publication de la demande: 29.05.2002
(73) Titulaire: Rumpler-Technologies, 93120 La Courneuve (FR)
(72) Inventeur: Courteix, Serge, 95280 Jouy Le Moutier (FR)
(74) Mandataire: Cardy, Sophie

(56) Documents cités:
- EP-A- 0 229 204
- EP-A- 0 240 787
- EP-A- 0 592 814
- FR-A- 2 777 787
- US-A- 4 986 818

## Description

L'invention concerne un dispositif de protection pour aiguille de seringue comportant un capuchon élastique d'aiguille.

Plus précisément, la présente invention concerne un dispositif de protection pour aiguille de seringue du type qui comporte un capuchon élastique d'aiguille de direction générale longitudinale possédant une extrémité distale fermée et une extrémité proximale ouverte, ledit capuchon étant formé d'une paroi latérale, s'étendant depuis ladite extrémité proximale le long d'une zone d'extrémité proximale en délimitant un logement interne destiné à recevoir la portion distale du corps d'une seringue à aiguille, et d'une paroi d'extrémité dont l'épaisseur s'étend depuis ladite extrémité distale le long d'une zone d'extrémité distale, ladite paroi d'extrémité étant susceptible d'être percée sur une partie de son épaisseur par l'extrémité libre de ladite aiguille, le logement comprenant, depuis ladite extrémité proximale, une ouverture présentant un diamètre maximal, un premier tronçon de forme tronconique ou cylindrique de section circulaire, un deuxième tronçon cylindrique de section circulaire présentant un diamètre inférieur au diamètre maximal et destiné à loger la portion distale du corps de seringue qui porte ladite aiguille, et un troisième tronçon qui va en se rétrécissant depuis le deuxième tronçon jusqu'au fond dudit logement.

Des dispositifs de protection pour aiguille de seringue du type précité ont déjà été proposés.

Ainsi, le brevet EP 0 429 052 concerne un ensemble de blindage d'aiguille comprenant, comme il apparaît sur la figure 5, une gaine élastique d'aiguille similaire au capuchon élastique d'aiguille défini précédemment.

Toutefois, ce type de dispositif de protection pour aiguille présente un certain nombre d'inconvénients.

Le dispositif de protection pour aiguille de seringue objet de la présente invention est destiné à être monté sur une seringue hypodermique permettant l'injection d'un liquide médicamenteux chez un patient.

Que ces seringues hypodermiques soient préremplies ou bien qu'elles soient remplies par le personnel hospitalier juste avant la réalisation de l'injection, il est nécessaire que cette seringue reste stérile jusqu'à son utilisation.

Par exemple, lorsque la seringue est conditionnée en étant déjà remplie de liquide, sont réalisées les différentes étapes suivantes pour la préparation de la seringue avant son conditionnement. En premier lieu, le corps de la seringue, sur la partie distale duquel est montée une aiguille enduite d'un revêtement de silicone, est lavé. Ensuite, un capuchon élastique d'aiguille est monté sur l'aiguille pour former un ensemble qui sera ultérieurement rendu stérile, de préférence par un passage en autoclave.

Après le passage en autoclave de l'ensemble précité, la seringue est remplie du liquide qui lui est destiné et le corps de seringue est refermé par le piston et le poussoir qui complètent la seringue avant son conditionnement ultérieur.

Pendant le passage en autoclave, du fait que le capuchon élastique d'aiguille est réalisé dans une matière permettant le passage des gaz (généralement du caoutchouc), il est possible de réaliser un équilibre de pression entre l'extérieur du capuchon élastique, c'est-à-dire l'enceinte de l'autoclave, et l'intérieur du capuchon élastique, c'est-à-dire le logement recevant l'aiguille de la seringue.

Pendant le cycle de stérilisation en autoclave, outre une augmentation de la température dans l'enceinte de l'autoclave, on réalise également de manière classique, après un ou plusieurs vides partiels dans l'enceinte, une augmentation importante de la pression (par exemple jusqu'à 2,3 bars). Cette pression maximale est maintenue durant un certain temps (plateau de pression) avant la réalisation d'un nouveau vide partiel dans l'enceinte de l'autoclave. A la fin du cycle, la pression est augmentée jusqu'à la pression atmosphérique, tandis que la température redescend progressivement jusqu'à la température ambiante.

Des explications précédentes, on comprend que pendant le cycle de stérilisation en autoclave, il existe des changements de pression assez brutaux dans l'enceinte. Ainsi, lors de la chute brutale de la pression dans l'enceinte entre la valeur maximale de pression et le vide partiel, il arrive que la pression dans le logement du capuchon élastique ne puisse pas s'équilibrer de manière assez rapide de sorte qu'il en résulte momentanément une pression résiduelle dans ce logement qui présente une valeur supérieure à celle de la pression régnant dans l'enceinte.

Dans certains cas, notamment lorsque la paroi latérale du capuchon élastique n'est pas suffisamment résistante, la pression résiduelle présente dans le logement engendre une déformation de cette paroi latérale qui peut conduire au déplacement relatif du capuchon élastique par rapport à la portion distale du corps de seringue sur laquelle est monté le capuchon. Ce déplacement du capuchon peut même être si important qu'il peut conduire à une séparation entre le capuchon et le corps de seringue lorsque la déformation subie par le capuchon ne permet pas de retenir la portion distale du corps de seringue dans le logement.

Dans le cas du déplacement entre le capuchon et le corps de seringue, il peut exister une rupture d'étanchéité entre le logement du capuchon et l'environnement extérieur au capuchon, ce qui induit un risque de perte de stérilité dans ce logement, donc de l'aiguille. La perte de stérilité devient avérée dans le cas de la séparation entre le capuchon et le corps de seringue.

Ce déplacement induit aussi un risque de fuite de liquide hors de la seringue d'où une dose de liquide dans la seringue dont le volume a diminué et un risque de contact avec ce liquide pour l'utilisateur, ce qui peut s'avérer dangereux dans le cas de certains liquide utilisés pour des examens, notamment en imagerie médicale. Egalement, la mise en contact de l'extrémité libre de l'aiguille avec l'environnement extérieur peut engendrer des réactions physico-chimiques sur le liquide, telles que cristallisation ou coagulation, susceptibles de dégrader ce liquide et de rendre la seringue inutilisable.

Ce risque de perte de stérilité est encore plus important dans le cas où le corps de seringue est réalisé en verre, car l'utilisation de ce matériau entraîne une plage de tolérance dimensionnelle beaucoup plus étendue que dans le cas d'une seringue réalisée en matière plastique.

La présente invention a pour objectif de fournir un dispositif de protection pour aiguille de seringue ne présentant pas les inconvénients précités, c'est-à-dire garantissant le maintien de l'aiguille dans une atmosphère stérile fermée d'une manière étanche jusqu'à son utilisation tout en conservant, voire en améliorant la facilité de retrait du dispositif de protection par l'utilisateur avant l'utilisation de la seringue.

Cet objectif est atteint par un dispositif de protection pour aiguille de seringue du type précité qui se caractérise en ce que la paroi latérale du capuchon est en outre munie d'un bourrelet annulaire disposé dans ledit logement entre lesdits premier et deuxième tronçons dudit logement, au moins une fente s'étendant en direction longitudinale sur ledit bourrelet annulaire.

On comprend que ce bourrelet annulaire permet de retenir la partie distale du corps de seringue dans le logement du capuchon, surtout lorsqu'il existe une pression résiduelle à l'intérieur de ce logement, le bourrelet annulaire constituant alors un moyen de retenue mécanique empêchant un déplacement relatif en translation longitudinale entre le capuchon et la portion distale du corps de seringue .Grâce à cette (ces) fente(s), on facilite le passage des gaz sous pression (en particulier de la vapeur d'eau sous pression utilisée pendant le passage en autoclave et qui réalise la stérilisation) entre le logement du capuchon et l'extérieur du capuchon. De cette manière, on obtient également une plus grande déformabilité du bourrelet annulaire dans le logement, ce qui permet de minimiser la force d'arrachement nécessaire lors de la séparation entre le capuchon et le corps de seringue.

Ce bourrelet annulaire est d'autant plus efficace pour retenir dans le logement la portion distale du corps de seringue, que cette partie distale est la plupart du temps réalisée sous la forme d'un renflement pouvant présenter une forme générale de boule ou de tronçon de sphère. Ce renflement présente, dans sa partie médiane, un diamètre plus grand que la partie de révolution, souvent tronconique, qui lui est adjacente. Ainsi, le bourrelet annulaire vient naturellement se loger dans la dépression annulaire formée sur la portion distale du corps de seringue, entre le renflement formant la partie médiane de la portion distale et la partie de révolution qui est adjacente à la portion distale.

Selon une caractéristique avantageuse, ledit bourrelet présente en section longitudinale une forme de demi goutte d'eau dont la partie la plus épaisse est tournée en direction de l'extrémité proximale du capuchon. Cette forme est particulièrement adaptée pour retenir la portion distale du corps de seringue dans le logement pendant le passage en autoclave tout en permettant également une séparation aisée entre le capuchon et le corps de l'aiguille, cette étape de séparation précédant l'utilisation de la seringue.

De préférence, la face extérieure de la paroi latérale qui entoure le bourrelet annulaire est en forme de tronc de cône.

Par ailleurs, il est prévu, selon un autre mode de réalisation de l'invention, que le dispositif de protection pour aiguille de seringue comporte en outre une coque rigide de forme générale longitudinale et cylindrique de section circulaire, présentant une extrémité proximale ouverte et une extrémité distale au moins partiellement fermée, ladite coque comprenant une paroi longitudinale s'étendant depuis ladite extrémité proximale jusqu'à ladite extrémité distale et une paroi terminale située à son extrémité distale, ladite coque étant destinée à entourer, en le contenant, ledit capuchon élastique d'aiguille et étant pourvue de moyens de retenue dudit capuchon.

L'utilisation supplémentaire d'une coque rigide entourant le capuchon élastique a principalement pour but de protéger toutes les personnes manipulant successivement la seringue, d'une piqûre accidentelle avec l'aiguille dans le cas où l'extrémité libre de l'aiguille serait amenée à percer complètement la paroi d'extrémité du capuchon élastique d'aiguille.

Cette coque rigide a également pour avantage de faciliter la manipulation du dispositif de protection pour aiguille de seringue par les machines des chaînes de fabrication et de montage, notamment lors du montage du dispositif de protection sur la seringue.

L'invention sera mieux comprise, et des caractéristiques secondaires et leurs avantages apparaîtront au cours de la description de modes de réalisation donnée ci-dessous à titre d'exemple.

Il est entendu que la description et les dessins ne sont donnés qu'à titre indicatif et non limitatif.

Il sera fait référence aux dessins annexés, dans lesquels :
- la figure 1 est une vue en coupe longitudinale diamétrale d'un dispositif de protection pour aiguille de seringue selon la présente invention comportant un capuchon élastique d'aiguille,
- la figure 2 est une élévation du dispositif de la figure 1 selon la direction II, c'est-à-dire depuis l'extrémité proximale du capuchon élastique d'aiguille,
- la figure 3 est une vue en élévation latérale partiellement transparente du dispositif de la figure 1,
- la figure 4 représente le dispositif de protection pour aiguille de la figure 1, disposé légèrement en retrait d'une partie distale de seringue composée de la portion distale du corps cylindrique de seringue et de l'aiguille,
- la figure 5 représente une vue en coupe longitudinale partielle du dispositif de protection pour aiguille monté sur la seringue,
- la figure 6 représente une vue en coupe longitudinale diamétrale d'une autre forme de réalisation du dispositif de protection pour aiguille comportant, outre le capuchon élastique d'aiguille, une coque rigide,
- la figure 7 est une vue en élévation depuis la direction VII de la figure 6, c'est-à-dire depuis l'extrémité distale du dispositif de protection pour aiguille,
- la figure 8 est une vue en élévation longitudinale du dispositif de protection de la figure 6,
- la figure 9 est une vue en perspective et en coupe selon la direction IX-IX de la figure 7 du dispositif de protection pour aiguille des figures 6 à 8, et
- la figure 10 est une vue en coupe longitudinale diamétrale du dispositif de protection pour aiguille de la figure 6 monté sur une seringue.

Dans la description qui suit de la présente invention, l'adjectif "distal" se rapporte à la partie la plus éloignée de la main de la personne tenant la seringue, et l'adjectif "proximal" se rapporte à la partie la plus proche de la main de la personne tenant la seringue.

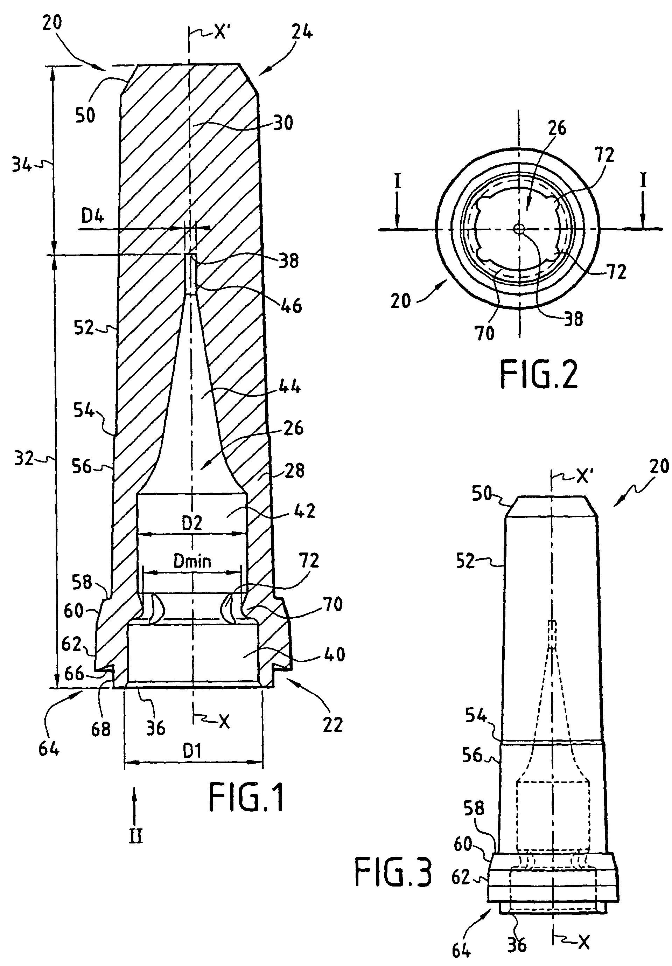

Sur les figures 1 à 5 est représenté un dispositif de protection pour aiguille de seringue composé d'un capuchon élastique d'aiguille 20 s'étendant selon une direction longitudinale (X, X') entre une extrémité proximale 22 ouverte et une extrémité distale 24 fermée. Le capuchon 20 définit un logement intérieur 26 délimité par une paroi latérale 28 et par une paroi d'extrémité 30.

La paroi latérale 28 s'étend, en direction longitudinale selon l'axe (X, X'), depuis l'extrémité proximale 22 le long d'une zone d'extrémité proximale 32 représentant environ les deux tiers de la longueur du capuchon 20.

La paroi d'extrémité 30 est donc pleine et s'étend, en direction longitudinale selon l'axe (X, X'), depuis l'extrémité distale 24 le long d'une zone d'extrémité distale 34 représentant environ un tiers de la longueur totale du capuchon 20. Ainsi, l'épaisseur de la paroi d'extrémité 30 (qui correspond à la longueur de la zone d'extrémité distale 34) permet le logement de l'extrémité libre de l'aiguille d'une seringue comme il sera expliqué ci-après.

Comme il est habituellement répandu pour ce type de dispositif de protection pour aiguille, le capuchon élastique 20 est de révolution autour de l'axe longitudinal (X, X'). Cette symétrie de révolution concerne le contour externe du capuchon 20 (paroi latérale 28 et paroi d'extrémité 30) ainsi que le contour interne de la paroi latérale 28 qui définit le logement 26, sauf en ce qui concerne la présence de fentes comme il sera expliqué ci-après.

Le logement interne 26 est composé de plusieurs tronçons s'étendant depuis une ouverture 36 située à l'extrémité proximale 22 du capuchon 20, jusqu'à un fond 38 situé de l'autre côté de la zone d'extrémité proximale 32.

De manière adjacente à l'ouverture 36, le logement 26 comporte un premier tronçon 40 qui présente une forme cylindrique de section circulaire d'axe (X, X') sur les figures, une forme tronconique allant en se rétrécissant en direction du fond 38 pouvant également être utilisée.

Tel qu'illustré, le premier tronçon 40 présente un diamètre sensiblement égal au diamètre D1 de l'ouverture 36.

De l'autre côté de l'ouverture 36, le premier tronçon précité 40 est voisin d'un deuxième tronçon 42 destiné à recevoir la portion distale d'un corps de seringue qui porte l'aiguille, ce deuxième tronçon présentant une forme cylindrique de section circulaire et d'axe longitudinale (X, X') et présentant un diamètre D2 inférieur au diamètre D1 de l'ouverture 36.

En direction opposée au premier tronçon 40, le deuxième tronçon 42 est prolongé par un troisième tronçon 44 dont le diamètre va en se rétrécissant progressivement en direction du fond 38 du logement 26. Au niveau du fond 38 du logement 26, est constitué un quatrième tronçon 46 cylindrique de section circulaire présentant un diamètre D4 sensiblement égal au diamètre de l'aiguille de seringue.

Le contour extérieur du capuchon 20 présente une forme générale cylindrique de section circulaire avec des variations de diamètre et de forme telles qu'exposées ci-après.

A partir de l'extrémité distale 24, au niveau de laquelle la face de la paroi d'extrémité 30 tournée vers l'extérieur est sensiblement plane, s'étend une première portion 50 en forme de tronc de cône d'étendue relativement limitée, une deuxième portion 52 de forme cylindrique de section circulaire légèrement évasée qui s'étend sensiblement jusqu'à la moitié de la zone d'extrémité proximale 32 à la hauteur du troisième tronçon 44 du logement 26.

La forme légèrement évasée de la deuxième portion 52 permet de manière classique de faciliter le démoulage du capuchon et la forme de la première portion 50 facilite le centrage et le montage de la coque rigide sur le capuchon comme il sera expliqué plus loin.

Un épaulement saillant 54 relie la deuxième portion 52 à une troisième portion 56 de forme cylindrique de section circulaire très légèrement évasée qui s'étend jusqu'au deuxième tronçon 42 du logement 26 à proximité du premier tronçon 40.

Un autre épaulement saillant 58 relie la troisième portion 56 à une quatrième portion 60 de forme tronconique qui s'étend jusqu'à la hauteur du premier tronçon 40 du logement 26. Cette quatrième portion 60 est prolongée en direction de l'extrémité proximale du capuchon 20 par une cinquième portion 62 de forme cylindrique de section circulaire, elle même adjacente à une sixième portion 64 située en retrait par rapport à la cinquième portion 62. Cette sixième portion 64 comporte une surface d'épaulement 66 formant un angle très légèrement inférieur à 90° par rapport à la cinquième portion 62 et une face annulaire 68 voisine de l'ouverture 36 et de forme cylindrique de section circulaire. Ainsi, cette sixième portion est constituée d'un épaulement annulaire 64 rentrant en direction de l'extrémité proximale 22.

Selon une caractéristique essentielle de la présente invention, entre les premier et deuxième tronçons 40 et 42 du logement 26, est prévu un bourrelet annulaire 70 formant un renflement interne de matière au niveau de l'extrémité du deuxième tronçon 42 tournée en direction de l'extrémité proximale 22.

De façon avantageuse, ce bourrelet 70 présente, en section longitudinale une forme de demi goutte d'eau dont la partie la plus épaisse est tournée en direction de l'extrémité proximale 22 du capuchon 20, la pointe de la goutte d'eau rejoignant le deuxième tronçon 42 en regard de l' autre épaulement saillant 58.

Ce bourrelet 70 délimite un contour intérieur du logement en forme de demi-poire et constitue un cordon de retenue mécanique pour la portion distale du corps de la seringue comme il sera expliqué ci-après. Lors du passage en autoclave, le bourrelet 70 garantit la retenue de la portion distale 104 de la seringue 100 dans le logement 26 ; même pour un écart de pression important entre le logement 26 et l'enceinte de l'autoclave. C'est seulement à partir d'une différence de pression forte ΔP1 (valeur positive et égale à l'écart entre la pression régnant dans le logement 26 et la pression régnant l'enceinte de l'autoclave) que la résistance mécanique du bourrelet 70 n'est pas suffisante et risquerait un déplacement relatif entre la seringue et le dispositif de protection d'aiguille.

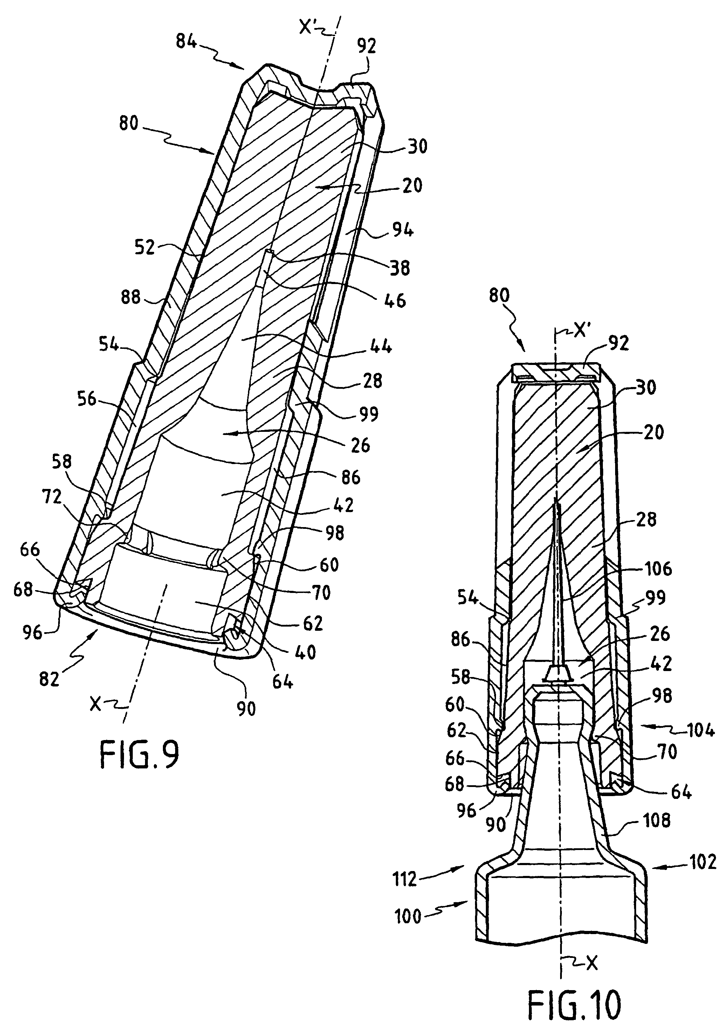

Afin de faciliter, lors du passage en autoclave, le passage de la vapeur d'eau sous pression hors du logement 26 à partir d'une certaine différence de pression plus faible (ΔP2<ΔP1) et d'améliorer la déformabilité de ce bourrelet annulaire 70, celui-ci est muni de quatre fentes 72 s'étendant en direction longitudinale sur le bourrelet 70, ces quatre fentes étant régulièrement angulairement réparties et ne s'étendant que sur une partie de l'épaisseur en direction radiale du bourrelet 70 comme on peut le voir sur la figure 2. De préférence (voir figure 9), les fentes 72 s'étendent radialement sur une profondeur du bourrelet 70 engendrant un diamètre entre deux fentes égal à D2.

On peut bien entendu prévoir de une à n fente(s) 72, lesquelles peuvent présenter une profondeur plus ou moins importante que le cas illustré à la figure 9. Si elles sont en grand nombre, ces fentes 72 séparent entre eux un grand nombre de portions du bourrelet 70 qui forment chacun une petite protubérance. De même, ces fentes 72 peuvent être plus ou moins larges que le cas illustré à la figure 9.

On peut bien entendu prévoir (cas de figure non représenté) un bourrelet qui présente une autre forme, en particulier qui n'est pas annulaire. Par exemple, entre deux fentes 72, le bourrelet peut présenter un contour interne renflé en forme d'un tronc de tore dont le centre est à l'extérieur du capuchon 20.

De cette manière, on constate qu'au niveau des premier et deuxième tronçons 40 et 42, le diamètre du logement 26 est minimal à l'emplacement du bourrelet 70 et présente une valeur Dₘᵢₙ.

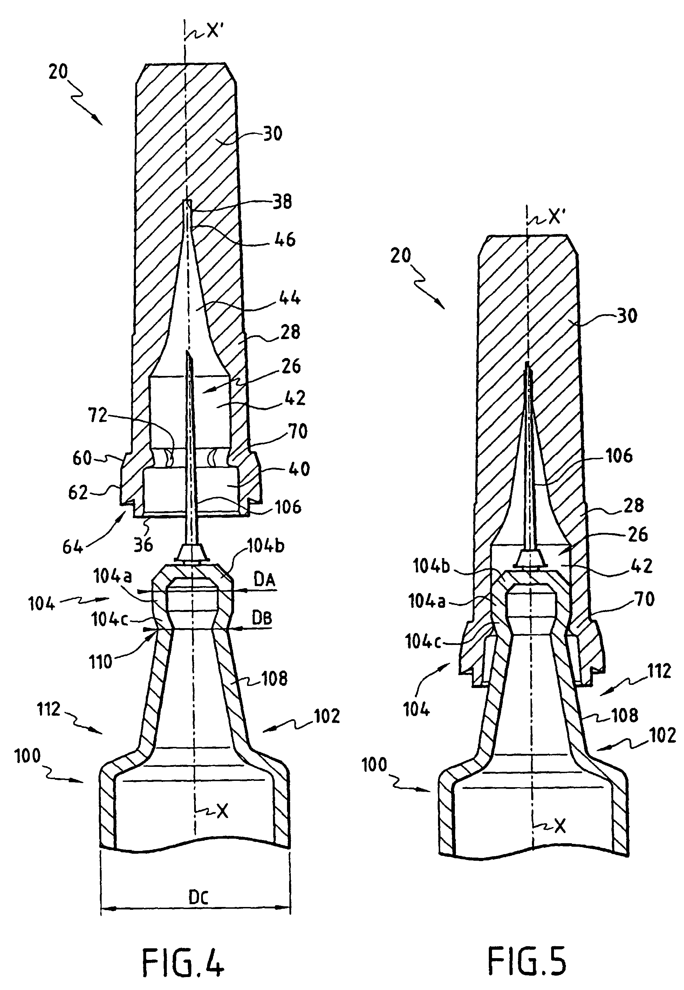

Comme on peut le voir sur les figures 4 et 5 représentant partiellement une seringue 100 hypodermique, le corps 102 cylindrique de cette seringue est muni d'une portion distale 104 qui porte l'aiguille 106.

Cette portion distale 104 présente une forme générale de sphère en constituant une boule portant l'aiguille 106. Plus précisément, la portion distale 104 forme un renflement annulaire formé de trois parties : une partie tronconique terminale 104b allant en se rétrécissant en direction de l'extrémité libre de l'aiguille 106, une partie médiane 104a en forme de cylindre de section circulaire de diamètre D_{A} et une partie tronconique de liaison 104c. La partie tronconique de liaison 104c est adjacente à une partie de révolution 108 en formant une dépression annulaire 110 (de diamètre D_{B}) par rapport à la partie la plus large de la portion distale 104.

Comme illustré sur les figures 4 et 5, lorsque l'aiguille 106 pénètre à l'intérieur du capuchon 20, l'extrémité libre de l'aiguille 106 vient se planter dans la paroi d'extrémité 30 du capuchon 20 tandis que la portion distale 104 du corps 102 de la seringue pénètre dans le logement 26 du capuchon 20 au niveau du deuxième tronçon 42.

Comme on peut le voir sur la figure 5, après montage, le bourrelet annulaire 70 est situé contre la partie tronconique de liaison 104c, ce qui réalise une retenue mécanique efficace de la partie distale 112 de la seringue 100, formée de l'aiguille 106 et du corps cylindrique 102, à l'intérieur du logement 26.

Du fait de la présence de ce bourrelet annulaire 70 qui vient s'appliquer en contact de manière serrée contre la partie tronconique de liaison 104c, il n'est pas nécessaire que le diamètre D2 du deuxième tronçon 42 du logement 26 assure un serrage important autour de la partie médiane 104a.

Toutefois, un contact étanche doit être assuré par le deuxième tronçon 42 du logement 26 contre la partie médiane 104a de la portion distale 104 de la seringue afin de conserver stérile le logement 26 après le passage en autoclave. L'étanchéité dont il est question ici est une étanchéité microbiologique permettant de conserver une stérilité, c'est-à-dire de garantir l'absence de germes microbiens ou de produits toxiques d'origine microbienne ou fongique.

De préférence, le diamètre D2 du deuxième tronçon 42 du logement 26 est supérieur ou égal à 85 %, de préférence sensiblement égal à 92 %, du diamètre extérieur D_{A} de la portion distale 104 du corps de seringue 102.

De préférence également, le diamètre Dₘᵢₙ du bourrelet annulaire 70 est compris entre 85 et 95 % du diamètre D2 du deuxième tronçon 42 du logement 26, le diamètre minimal Dₘᵢₙ étant de préférence sensiblement égal à 90 % du diamètre D2 du deuxième tronçon 42.

En particulier, on a réalisé des essais avec une seringue 100 de 1 ml présentant un corps de seringue 102 de diamètre extérieur D_{C} = 8,15 mm, le diamètre extérieur D_{A} de la zone sphérique 104a étant égal à 4,35 mm, tandis que le diamètre extérieur D_{B} de la zone rétrécie 104c est égal à 3,85 mm.

Pour ce type de seringue, le capuchon 20 utilisé présente alors les dimensions suivantes :
- D2 (diamètre du deuxième tronçon 42 du logement 26) : 4 mm,
- Dₘᵢₙ (diamètre du logement 26 au niveau du bourrelet annulaire 70) : 3,6 mm,
- D1 (diamètre de l'ouverture 36 du logement 26) : 4,7 mm.

Les dimensions qui précèdent sont données pour un capuchon 20 présentant une longueur totale de 23,5 mm pour un diamètre extérieur de 7 mm au niveau de la cinquième portion 62, la quatrième portion 60 allant en se rétrécissant jusqu'à un diamètre extérieur minimal de 6,5 mm.

Avec un tel capuchon, on obtient une étanchéité du logement 26 qui est maintenue pendant tout le cycle de stérilisation en autoclave sans aucun déplacement entre le capuchon 20 et la partie distale 104 de la seringue.

En effet, le bourrelet annulaire 70 assure le blocage mécanique de la portion distale 104 de la seringue dans le deuxième tronçon 42 du logement 26 pendant le passage en autoclave. En outre, le contact étanche assuré par le deuxième tronçon 42 du logement 26 contre la partie médiane 104a de la portion distale 104 de la seringue engendre une étanchéité microbiologique entre le logement 26 et l'extérieur du dispositif de protection pendant et après le passage en autoclave, ce qui garantit une stérilité permanente du logement et de l'aiguille jusqu'à l'utilisation de la seringue 100, plus précisément jusqu'à la séparation entre le capuchon 20 et l'aiguille 106.

En outre, ce mode de réalisation permet d'obtenir, quel que soit l'état de départ de l'aiguille (sèche, mouillée, siliconée), une résistance à l'effort pour séparer le capuchon 20 de la seringue 100 qui est de l'ordre de 9 N, ce qui garantit un confort d'utilisation à l'ouverture, cette valeur étant plus faible que pour les produits de l'art antérieur et en tout cas plus faible que la force d'arrachement maximale admissible qui est de l'ordre de 35 N.

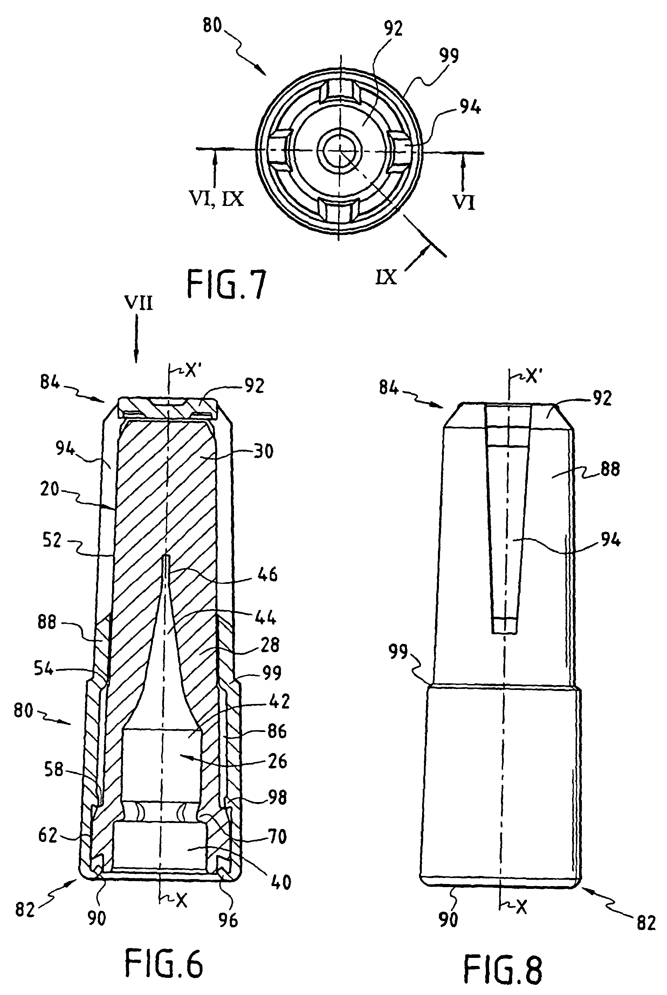

Comme il est illustré sur les figures 6 à 10, la présente invention concerne également un dispositif de protection pour aiguille de seringue qui comporte outre le capuchon élastique 20 présenté précédemment, une coque rigide 80 dans laquelle est logé le capuchon 20.

Ce type de coque 80 est classiquement utilisé pour renforcer la protection de l'utilisateur de la seringue contre une piqûre par aiguille en offrant une protection supplémentaire extérieure rigide qui est difficilement percée par l'aiguille 106.

Cette coque rigide 80 présente une forme générale longitudinale cylindrique de section circulaire, elle est montée de manière coaxiale par rapport au capuchon 20 et elle s'étend entre une extrémité proximale 82 ouverte et une extrémité distale 84 fermée.

La coque rigide 80 est dimensionnée pour permettre l'insertion et le blocage du capuchon 20 à l'intérieur. A cet effet, la cavité 86 délimitée par le contour intérieur de la coque rigide 80 présente une forme qui suit sensiblement la forme extérieure du capuchon 20.

La coque rigide 80 est composée d'une paroi longitudinale 88 qui s'étend depuis l'extrémité proximale 82 au niveau de l'ouverture 90 jusqu'à l'extrémité distale 84 au niveau de laquelle la paroi longitudinale 88 se prolonge par une paroi terminale 92 qui referme la cavité 86.

La paroi longitudinale 88 est munie, entre la paroi terminale 92 et environ deux cinquièmes de la longueur de la paroi longitudinale, de quatre découpes 94 destinées à permettre le passage de la vapeur d'eau sous pression depuis l'enceinte de l'autoclave jusqu'au logement 26.

Ces quatre découpes 94 sont (voir figures 7 et 8) de forme générale longitudinale et elles sont réparties radialement à 90° l'une de l'autre.

Afin de retenir le capuchon 20 à l'intérieur de la cavité 86 de la coque rigide 80, sont prévus des moyens de retenue du capuchon comprenant un rebord rentrant 96, de préférence annulaire, qui forme un élément, de préférence une collerette, faisant saillie vers l'intérieur de la cavité 86.

Ce rebord rentrant 96 est donc logé dans le décrochement formé par la sixième portion 64 du contour extérieur du capuchon 20 de sorte que le capuchon 20 est empêché de sortir de la coque 80 par la venue en butée axiale du rebord rentrant 96 sur la surface d'épaulement 66.

Lors du montage, lorsque le capuchon 20 est enfoncé dans la coque 80 au maximum, la face extérieure essentiellement plane de la paroi d'extrémité 30 du capuchon 20 vient en butée axiale contre la face intérieure de la paroi terminale 92 de la coque 80. Par contre, en position normale, il n'y a pas de contact entre la face extérieure de la paroi d'extrémité 30 du capuchon 20 et la face intérieure de la paroi terminale 92 de la coque 80.

En outre, afin de compléter le blocage du mouvement relatif en translation longitudinale entre le capuchon 20 et la coque rigide 80, plus particulièrement le mouvement du capuchon 20 en direction de la paroi terminale 92 de la coque 80, est prévue, comme moyen de retenue complémentaire, une nervure annulaire 98 disposée sur la face interne de la paroi longitudinale 88 de la coque 80. Cette nervure annulaire 98 est apte à coopérer avec la face extérieure de la paroi latérale 28 du capuchon 20 en venant en butée contre l'épaulement 58 du capuchon 20.

Cet épaulement 58 forme un premier épaulement rentrant en direction de l'extrémité distale 24 du dispositif, ce premier épaulement 58 étant situé sur la face extérieure de la paroi latérale 28 du capuchon 20 en regard du deuxième tronçon 42 du logement 26.

Comme on peut le voir sur la figure 6, la paroi longitudinale 88 comporte, environ à mi-longueur de la coque 80, au moins sur sa face externe, et de préférence (cas de figure illustré) sur toute l'épaisseur de la paroi longitudinale 88 de la coque 80, un épaulement 99 annulaire rentrant en direction de l'extrémité distale 24 du dispositif (le diamètre intérieur de la cavité 86 étant plus faible du côté de l'épaulement 99 situé le plus près de la paroi terminale 92).

Cet épaulement rentrant 99 est formé de façon à se retrouver en face de l'épaulement 54 du capuchon 20 qui forme un deuxième épaulement rentrant en direction de l'extrémité distale du dispositif en étant situé sur la face extérieure de la paroi latérale 28 du capuchon 20 en regard du troisième tronçon 44 du logement 26.

Comme on peut le voir sur les figures 7 et 8, l'épaulement rentrant 99 se trouve à la fois sur la face interne et sur la face externe de la paroi longitudinale 88 de façon à former un décrochement annulaire de cette paroi.

Cet épaulement 99 annulaire a notamment pour but de faciliter la manipulation du dispositif de protection d'aiguille par les différentes machines de la chaîne de fabrication et de montage.

Comme on peut le voir sur la figure 10, dans le cas où le capuchon 20 est contenu dans la coque rigide 80, le dispositif de protection ainsi formé peut être monté sur une seringue 100 de la même manière qu'avec le capuchon 20 seul.

En outre, il existe, lorsque le capuchon 20 est inséré à l'intérieur de la cavité 86 de la coque 80, un contact entre la cinquième portion 62 du contour extérieur du capuchon 20 et la face intérieure de la paroi longitudinale 88 située entre la nervure 98 et le rebord 96.

Au moment de l'insertion de la portion distale 104 du corps 102 de la seringue dans le logement 26, il y a un écrasement du bourrelet annulaire 70 par la portion distale 104 du corps 102 de la seringue. La paroi latérale 28 du capuchon étant réalisée dans une matière suffisamment souple (par exemple du caoutchouc), une partie de la déformation est absorbée par les fentes 72.

D'autre part, la paroi latérale 28 du capuchon présentant une épaisseur suffisamment mince au regard de la quatrième portion 60 du contour extérieur du capuchon 20, la déformation du bourrelet annulaire 70 engendre un écartement radial de la paroi latérale 28 à cet endroit d'où une déformation de la quatrième portion 60 qui se rapproche de la face interieure de la paroi longitudinale 88 cylindrique de la coque 80 : ceci est rendu possible grâce à la forme extérieure en tronc de cône de la quatrième portion 60 de la paroi latérale 28 qui se trouve en regard du bourrelet annulaire 70 et à l'espace annulaire disponible sur la face intérieure de la paroi longitudinale 88 de la coque 80 en regard de cette quatrième portion 60.

Il est entendu que la quatrième portion 60 pourrait également présenter une forme de tronc de cône inversée par rapport à celle illustrée, c'est-à-dire une forme allant en se rétrécissant en direction de l'extrémité proximale 22 du capuchon.

Cette quatrième portion 60 pourrait aussi présenter une forme différente de celle d'un tronc de cône, pourvu que cette surface engendre, une fois que le capuchon est logé dans la coque, un espace annulaire libre entre elle et la zone qui lui fait face de la paroi longitudinale 88 de la coque rigide 80. En effet, cet espace annulaire permet de recevoir une partie de la déformation radiale vers l'extérieur de la zone de la paroi latérale 28 du capuchon 20 qui se trouve contre la partie médiane 104a de la portion distale 104 de la seringue (voir figure 10).

De manière plus générale, il existe de façon préférentielle un espace, avantageusement annulaire comme il est représenté sur les figures 6, 9 et 10, entre la paroi longitudinale 88 de la coque rigide 80 et la paroi latérale 28 du capuchon 20 qui s'étend de manière longitudinale selon l'axe (X, X') depuis les découpes 94 jusqu'à la nervure annulaire 98 disposée sur la face interne de la paroi longitudinale 88 de la coque 80, c'est-à-dire jusqu'à l'épaulement saillant 58, de part et d'autre de l'épaulement 99. Cet espace permet le passage de la vapeur d'eau sous pression depuis l'enceinte de l'autoclave jusqu'au logement 26 grâce à la perméabilité aux gaz, notamment à la vapeur d'eau sous pression, du matériau constituant le capuchon 20. De plus, la partie de cet espace qui entoure le deuxième tronçon 42 du logement 26 permet également de recevoir l'expansion radiale de la zone de la paroi latérale 28 du capuchon 20 qui entoure la partie médiane 104a de la portion distale 104 de la seringue (voir figure 10).

Ainsi, on comprend que la présence de la coque ne modifie en rien l'utilisation et le fonctionnement du capuchon 20 pour la stérilisation pendant le passage en autoclave et pour le maintien de la stérilité de la seringue 106 après le passage en autoclave. En outre, la présence de la coque ne modifie pas la résistance à l'effort pour séparer le capuchon 20 de la seringue 100, tout en augmentant la sécurité de l'utilisateur contre une piqure.

Bien entendu, d'autres formes que celles illustrées sur les figures sont possibles pour le capuchon 20 et pour la coque 80.

## Revendications

1. Dispositif de protection pour aiguille de seringue comportant un capuchon (20) élastique d'aiguille de direction générale longitudinale possédant une extrémité distale (24) fermée et une extrémité proximale (22) ouverte, ledit capuchon (20) étant formé d'une paroi latérale (28), s'étendant depuis ladite extrémité proximale (22) le long d'une zone d'extrémité proximale (32) en délimitant un logement interne (26) destiné à recevoir la portion distale (104) du corps (102) d'une seringue à aiguille (100), et d'une paroi d'extrémité (30) dont l'épaisseur s'étend depuis ladite extrémité distale (24) le long d'une zone d'extrémité distale (34), ladite paroi d'extrémité (30) étant susceptible d'être percée sur une partie de son épaisseur par l'extrémité libre de ladite aiguille (106),
ledit logement (26) comprenant, depuis ladite extrémité proximale (22) :
- une ouverture (36) présentant un diamètre maximal (D1),
- un premier tronçon (40) de forme tronconique ou cylindrique de section circulaire,
- un deuxième tronçon (42) cylindrique de section circulaire présentant un diamètre (D2) inférieur audit diamètre maximal (D1) et destiné à loger la portion distale (104) du corps de seringue (102) qui porte ladite aiguille (106), et
- un troisième tronçon (44) qui va en se rétrécissant depuis ledit deuxième tronçon (42) en direction du fond (38) dudit logement (26),
**caractérisé en ce que** ladite paroi latérale (28) est en outre munie d'un bourrelet annulaire (70) disposé dans ledit logement (26) entre lesdits premier et deuxième tronçons (40, 42) dudit logement (26), au moins une fente (72) s'étendant en direction longitudinale sur ledit bourrelet annulaire (70).

2. Dispositif selon la revendication 1, **caractérisé en ce que** ledit bourrelet (70) présente en section longitudinale une forme de demi goutte d'eau dont la partie la plus épaisse est tournée en direction de l'extrémité proximale (22) du capuchon (20).

3. Dispositif selon la revendication 1 ou 2, **caractérisé en ce qu'**au niveau des premier et deuxième tronçons (40, 42) le diamètre du logement (26) est minimal à l'emplacement dudit bourrelet (70), ledit diamètre minimal (Dₘᵢₙ) étant compris entre 85 et 95 % dudit diamètre (D2) dudit deuxième tronçon (42).

4. Dispositif selon la revendication 3, **caractérisé en ce que** ledit diamètre minimal (Dₘᵢₙ) est sensiblement égal à 90% dudit diamètre (D2) dudit deuxième tronçon (42).

5. Dispositif selon la revendication 1, **caractérisé en ce que** ledit capuchon (20) est de révolution autour d'un axe longitudinal (X, X').

6. Dispositif selon l'une quelconque des revendications précédentes, **caractérisé en ce que** quatre fentes (72) régulièrement angulairement réparties s'étendent en direction longitudinale sur ledit bourrelet annulaire (70).

7. Dispositif selon l'une quelconque des revendications précédentes, **caractérisé en ce que** ledit diamètre (D2) dudit deuxième tronçon (42) est supérieur ou égal à 85% du diamètre extérieur (D_{A}) de la portion distale (104) du corps de seringue (102).

8. Dispositif selon l'une quelconque des revendications précédentes, **caractérisé en ce que** ledit diamètre (D2) dudit deuxième tronçon (42) est sensiblement égal à 92% du diamètre extérieur (D_{A}) de la portion distale (104) du corps de seringue (102).

9. Dispositif selon l'une quelconque des revendications précédentes, **caractérisé en ce que** la face extérieure (60) de la paroi latérale qui entoure ledit bourrelet annulaire (70) est en forme de tronc de cône.

10. Dispositif selon l'une quelconque des revendications précédentes, **caractérisé en ce qu'**il comporte en outre une coque rigide (80) de forme générale longitudinale et cylindrique de section circulaire présentant une extrémité proximale (82) ouverte et une extrémité distale (84) au moins partiellement fermée, ladite coque (80) comprenant une paroi longitudinale (88) s'étendant depuis ladite extrémité proximale (82) jusqu'à ladite extrémité distale (84) et une paroi terminale (92) située à son extrémité distale, ladite coque (80) étant destinée à entourer en le contenant ledit capuchon (20) élastique d'aiguille et étant pourvue de moyens de retenue (96, 98, 99) dudit capuchon (20).

11. Dispositif selon la revendication 10, **caractérisé en ce que** lesdits moyens de retenue comportent un rebord annulaire rentrant (96) formant l'extrémité libre proximale de la coque (80) et présentant un diamètre intérieur apte à retenir dans la coque (80) l'extrémité proximale de la paroi latérale (28) du capuchon (20).

12. Dispositif selon la revendication 11, **caractérisé en ce que** l'extrémité proximale de la surface extérieure de la paroi latérale (28) du capuchon (20) comporte un épaulement annulaire rentrant (64) en direction de l'extrémité proximale (22) du dispositif et apte à coopérer avec ledit rebord annulaire (96).

13. Dispositif selon la revendication 11, **caractérisé en ce que** lesdits moyens de retenue comportent en outre une nervure annulaire (98) disposée sur la face interne de la paroi longitudinale (88) de la coque (80) et apte à coopérer avec un premier épaulement rentrant (58) en direction de l'extrémité distale (24) du dispositif et situé sur la surface extérieure de la paroi latérale (28) du capuchon (20) en regard du deuxième tronçon (42) dudit logement (26).

14. Dispositif selon l'une quelconque des revendications 10 à 13, **caractérisé en ce que** la paroi longitudinale de la coque (80) présente, au moins sur sa face externe, un épaulement annulaire rentrant (99) en direction de l'extrémité distale (24) du dispositif.

## Patentansprüche

1. Schutzvorrichtung für eine Spritzennadel mit einer elastischen Nadelkappe (20) mit allgemeiner Längsrichtung, welche ein geschlossenes distales Ende (24) und ein offenes proximales Ende (22) besitzt,
wobei die Kappe (20) von einer Seitenwand (28), die sich von dem proximalen Ende (22) unter Begrenzung einer Innenaufnahme (26) zur Aufnahme des distalen Teils (104) des Körpers (102) einer Nadelspritze (100) entlang eines proximalen Endbereichs (32) erstreckt, sowie von einer Endwand (30) gebildet ist, deren Dicke sich von dem genannten distalen Ende (24) entlang eines distalen Endbereichs (34) erstreckt,
wobei die Endwand (30) geeignet ist, über einen Teil ihrer Dicke von dem freien Ende der Nadel (106) durchstochen zu werden,
wobei die Aufnahme (26) von dem proximalen Ende (22) aus folgendes umfaßt:
- eine Öffnung (36), die einen maximalen Durchmesser (D1) aufweist,
- einen ersten kegelstumpfförmigen oder zylindrischen Abschnitt (40) mit kreisförmigem Querschnitt,
- einen zweiten zylindrischen Abschnitt (42) mit kreisförmigem Querschnitt, der einen Durchmesser (D2), welcher kleiner ist als der maximale Durchmesser (D1), aufweist und dazu bestimmt ist, den distalen Teil (104) des Spritzenkörpers (102), welcher die Nadel (106) trägt, aufzunehmen, sowie
- einen dritten Abschnitt (44), der sich von dem zweiten Abschnitt (42) in Richtung auf den Boden (38) der Aufnahme (26) verjüngt,
**dadurch gekennzeichnet,**
**daß** die Seitenwand (28) ferner mit einem ringförmigen Wulst (70) versehen ist, welcher in der Aufnahme (26) zwischen dem ersten und dem zweiten Abschnitt (40, 42) der Aufnahme (26) angeordnet ist, wobei sich wenigstens ein Schlitz (72) in Längsrichtung über den ringförmigen Wulst (70) erstreckt.

2. Vorrichtung nach Anspruch 1, **dadurch gekennzeichnet, daß** der genannte Wulst (70) im Längsschnitt die Form eines halben Wassertropfens aufweist, dessen dickster Teil dem proximalen Ende (22) der Kappe (20) zugewandt ist.

3. Vorrichtung nach Anspruch 1 oder 2, **dadurch gekennzeichnet, daß** im Bereich des ersten und des zweiten Abschnitts (40, 42) der Durchmesser der Aufnahme (26) an der Stelle der Wulstes (70) minimal ist, wobei der minimale Durchmesser (Dₘᵢₙ) zwischen 85 und 95 % des Durchmessers (D2) des zweiten Abschnitts (42) beträgt.

4. Vorrichtung nach Anspruch 3, **dadurch gekennzeichnet, daß** der minimale Durchmesser (Dₘᵢₙ) im wesentlichen gleich 90 % des Durchmessers (D2) des zweiten Abschnitts (42) beträgt.

5. Vorrichtung nach Anspruch 1, **dadurch gekennzeichnet, daß** sich die Kappe (20) um eine Längsachse (X, X') dreht.

6. Vorrichtung nach irgendeinem der vorhergehenden Ansprüche, **dadurch gekennzeichnet, daß** sich vier winkelig gleichmäßig verteilte Schlitze (72) in Längsrichtung über den ringförmigen Wulst (70) erstrecken.

7. Vorrichtung nach irgendeinem der vorhergehenden Ansprüche, **dadurch gekennzeichnet, daß** der Durchmesser (D2) des zweiten Abschnitts (42) größer oder gleich 85 % des Außendurchmessers (D_{A}) des distalen Teils (104) des Spritzenkörpers (102) ist.

8. Vorrichtung nach irgendeinem der vorhergehenden Ansprüche, **dadurch gekennzeichnet, daß** der Durchmesser (D2) des zweiten Abschnitts (42) im wesentlichen gleich 92 % des Außendurchmessers (D_{A}) des distalen Teils (104) des Spritzenkörpers (102) ist.

9. Vorrichtung nach irgendeinem der vorhergehenden Ansprüche, **dadurch gekennzeichnet, daß** die Außenseite (60) der Seitenwand, welche den ringförmigen Wulst (70) umgibt, kegelstumpfförmig ausgebildet ist.

10. Vorrichtung nach irgendeinem der vorhergehenden Ansprüche, **dadurch gekennzeichnet, daß** sie außerdem eine steife Schale (80) von allgemein länglicher und zylindrischer Form mit kreisförmigem Querschnitt umfaßt, die ein offenes proximales Ende (82) und ein wenigstens teilweise geschlossenes distales Ende (84) aufweist, wobei die Schale (80) eine Längswand (88), die sich von dem proximalen Ende (82) bis zu dem distalen Ende (84) erstreckt, sowie eine an ihrem distalen Ende befindliche Endwand (92) aufweist, wobei die Schale (80) dazu bestimmt ist, die elastische Nadelkappe (20) enthaltend zu umschließen und mit Mitteln (96, 98, 99) zum Halten der Kappe (20) versehen ist.

11. Vorrichtung nach Anspruch 10, **dadurch gekennzeichnet, daß** die genannten Haltemittel einen ringförmigen, einspringenden Rand (96) aufweisen, welcher das freie proximale Ende der Schale (80) bildet und einen Innendurchmesser aufweist, der geeignet ist, das proximale Ende der Seitenwand (28) der Kappe (20) in der Schale (80) zu halten.

12. Vorrichtung nach Anspruch 11, **dadurch gekennzeichnet, daß** das proximale Ende der Außenfläche der Seitenwand (28) der Kappe (20) eine in Richtung des proximalen Endes (22) der Vorrichtung einspringende, ringförmige Schulter (64) aufweist, die geeignet ist, mit dem ringförmigen Rand (96) zusammenzuwirken.

13. Vorrichtung nach Anspruch 11, **dadurch gekennzeichnet, daß** die Haltemittel weiterhin eine ringförmige Nut (98) aufweisen, die an der Innenseite der Längswand (88) der Schale (80) angeordnet und geeignet ist, mit einer ersten, in Richtung des distalen Endes (24) der Vorrichtung einspringenden Schulter (58) zusammenzuwirken, die an der Außenfläche der Seitenwand (28) der Kappe (20) gegenüber dem zweiten Abschnitt (42) der Aufnahme (26) gelegen ist.

14. Vorrichtung nach irgendeinem der Ansprüche 10 bis 13, **dadurch gekennzeichnet, daß** die Längswand der Schale (80) wenigstens an ihrer Außenseite eine in Richtung des distalen Endes (24) der Vorrichtung einspringende, ringförmige Schulter (99) aufweist.

## Claims

1. Device for protecting a syringe needle comprising an elastic needle cap (20) of generally longitudinal direction presenting a closed distal end (24) and an open proximal end (22), said cap (20) being formed by a lateral wall (28) extending from said proximal end (22) along a proximal end zone (32), defining an inner housing (26) adapted to receive the distal part (104) of the body (102) of a needle syringe (100), and by an end wall (30) whose thickness extends from said distal end (24) along a distal end zone (34), said end wall (30) being capable of being pierced through a part of its thickness by the free end of said needle (106),
said housing (26) comprising, from said proximal end (22):
- an opening (36) presenting a maximum diameter (D1),
- a first portion (40) of truncated or cylindrical shape, of circular cross-section,
- a second cylindrical portion (42) of circular cross-section presenting a diameter (D2) smaller than said maximum diameter (D1) and intended to house the distal part (104) of the syringe body (102) which bears said needle (106), and
- a third portion (44) which narrows from said second portion (42) in the direction of the back (38) of said housing (26),
**characterised in that** said lateral wall (28) is further provided with an annular bead (70) disposed in said housing (26) between said first and second portions (40, 42) of said housing (26), at least one slot (72) extending longitudinally over said annular bead (70).

2. The device according to Claim 1, **characterised in that** said bead (70) presents in longitudinal cross-section the shape of a half drop of water of which the widest part faces the proximal end (22) of the cap (20).

3. The device according to Claim 1 or 2, **characterised in that**, at the level of the first and second portions (40, 42), the diameter of the housing (26) is minimum at the location of said bead (70), said minimum diameter (Dₘᵢₙ) being included between 85 and 95% of said diameter (D2) of said second portion (42).

4. The device according to claim 3, **characterised in that** said minimum diameter (Dₘᵢₙ) is substantially equal to 90% of said diameter (D2) of said second portion (42).

5. The device according to claim 1, **characterised in that** said cap (20) is of revolution about a longitudinal axis (X, X').

6. The device according to any one of the preceding claims, **characterised in that** four slots (72) regularly distributed angularly extend in the longitudinal direction over said annular bead (70).

7. The device according to any one of the preceding claims, **characterised in that** said diameter (D2) of said second portion (42) is greater than or equal to 85% of the outer diameter (D_{A}) of the distal part (104) of the syringe body (102).

8. The device according to any one of the preceding claims, **characterised in that** said diameter (D2) of said second portion (42) is substantially equal to 92% of the outer diameter (D_{A}) of the distal part (104) of the syringe body (102).

9. The device according to any one of the preceding claims, **characterised in that** the outer face (60) of the lateral wall which surrounds said annular bead (70) is in the form of a frustum of a cone.

10. The device according to any one of the preceding claims, **characterised in that** it further comprises a rigid shell (80) of general longitudinal and cylindrical shape, of circular cross-section, presenting an open proximal end (82) and an at least partially closed distal end (84), said shell (80) comprising a longitudinal wall (88) extending from said proximal end (82) up to said distal end (84) and a terminal wall (92) located at its distal end, said shell (80) being intended to surround and contain said elastic needle cap (20) and being provided with means (96, 98, 99) for retaining said cap (20).

11. The device according to claim 10, **characterised in that** said retaining means comprise a re-entrant annular flange (96) forming the proximal free end of the shell (80) and presenting an inner diameter adapted to retain in the shell (80) the proximal end of the lateral wall (28) of the cap (20).

12. The device according to claim 11, **characterised in that** the proximal end of the outer surface of the lateral wall (28) of the cap (20) comprises a re-entrant annular shoulder (64) in the direction of the proximal end (22) of the device and adapted to cooperate with said annular flange (96).

13. The device according to claim 11, **characterised in that** said retaining means further comprise an annular rib (98) disposed on the inner face of the longitudinal wall (88) of the shell (80) and adapted to cooperate with a first re-entrant shoulder (58) in the direction of the distal end (24) of the device and located on the outer surface of the lateral wall (28) of the cap (20) opposite the second portion (42) of said housing (26).

14. The device according to any one of claims 10 to 13, **characterised in that** the longitudinal wall of the shell (80) presents, at least on its outer face, a re-entrant annular shoulder (99) in the direction of the distal end (24) of the device.
